# EUROPEAN PATENT SPECIFICATION

(11) **EP 1 443 943 B1**
(45) Date of publication and mention of the grant of the patent: **27.10.2010**
(21) Application number: 02783017.3
(22) Date of filing: 28.10.2002
(51) Int. Cl.: A61K 31/675, C07F 9/564, C07F 9/62, C07D 455/02, C07G 5/00

(54) **PROCESS FOR REACTING ALKALOIDS AND USE OF THE REACTION PRODUCTS IN THE PREPARATION OF MEDICAMENTS**
VERFAHREN ZUR REAGIEREN VON ALKALOIDEN UND VERWENDUNG DER REAKTIONSPRODUCKTE IN DER BEREITSTELLUNG VON MEDIKAMENTEN
PROCESSUS DE REACTION D'ALCALOIDES ET UTILISATION DES PRODUITS DE REACTION DANS LA PREPARATION DE MEDICAMENTS

(30) Priority: 15.11.2001 CH 20942001
(43) Date of publication of application: 11.08.2004
(73) Proprietor: Nowicky, Wassyl, A-1040 Wien (AT)
(72) Inventor: Nowicky, Wassyl, A-1040 Wien (AT)
(74) Representative: Bogensberger, Burkhard
(86) International application number: PCT/EP2002/012003
(87) International publication number: WO 2003/041721

(56) References cited:
- WO-A-01/70203
- WO-A-95/25522
- AT-B- 354 644
- AT-B- 377 988
- FR-A- 2 366 020
- GB-A- 1 304 064
- GB-A- 2 110 533
- US-A- 4 970 212
- US-A- 5 981 512
- POTOPALSKY A I ET AL: "Semisynthetic antitumor alkaloids derivatives as a antiviral and a potential anti-HIV preparations" ANTIVIRAL RESEARCH, ELSEVIER SCIENCE BV., AMSTERDAM, NL, vol. 20, no. SUPPL 1, 1993, page 75 XP002046197 ISSN: 0166-3542
- JAGIELLO-WOJTOWICZ E ET AL: "Effect of six-month treatment with ukrain on early osteoporosis induced by ovariectomy in rats. Parts I-III. Preliminary studies of bone parameters, peripheral blood parameters and some hormone levels" DRUGS UNDER EXPERIMENTAL AND CLINICAL RESEARCH, vol. 22, no. 3-5, 1996, pages 173-184, XP009005271 ISSN: 0378-6501
- COLOMBO M L ET AL: "Pharmacological activities of chelidonium majus l. (papaveraceae)" PHARMACOLOGICAL RESEARCH, vol. 33, no. 2, 1996, pages 127-134, XP002230679 ISSN: 1043-6618

## Description

### Field of the invention

The invention relates to a novel process for reacting alkaloids with a phosphorus derivative and the use of the reaction products obtained.

### Background of the invention and prior art

Alkaloids are known for their biological activity in foreign organisms. For example, the milky sap of *Chelidonium majus L.* ("greater celandine") has long been known in popular medicine for the treatment of warts. *Chelidonium majus L.* contains more than 30 alkaloids, one of which is chelidonine, which accounts for up to 80% of the composition.

If a cytostatic and/or carcinogenic phosphorus derivative is added to alkaloids, synthesis products which are less toxic than the starting materials but have cytotoxic activity against certain cancer cell lines are obtained.

DE 2 028 330 and US 3 865 830 disclose the preparation of thiophosphoramide-isoquinoline adducts by reacting selected alkaloids of *Chelidonium majus L.* with tris(1-aziridinyl)phosphine sulphide in an organic solvent.

AT 354 644 and AT 377 988 describe processes for the preparation of phosphorus derivatives of alkaloids by reaction with carcinostatic phosphorus compounds, which are provided in a water-soluble form by conversion into their salt. A disadvantage of the disclosed processes is that the conversion of the reaction products into a water-soluble salt is not complete and the predominant part of the reaction products remains water-insoluble.

US 5 981 512 discloses the use of the substances disclosed in AT 377 988 and AT 354 644 for the treatment of radiation damage.

The compounds described in said patents have different cytostatic and carcinostatic activity. Mixtures of alkaloids, in particular of the total alkaloids of *Chelidonium majus L*., have proved therapeutically particularly promising, the pharmacological activity of which has been demonstrated in several studies on cancer treatment. It was found that the composition of the preparation is important for the clinical effect. According to the phytochemical and phytotherapeutic teaching, it is assumed that not only individual components but the totality of the mixture of the reacted alkaloids are or is pharmacologically active.

The residues of unreacted reagent after the reaction are removed from the synthesis mixture. Since tris(1-aziridinyl)phosphine sulphide is soluble in organic solvents, such as benzene, ether or chloroform, it is proposed in processes according to the prior art to remove the unreacted tris(1-aziridinyl)phosphine sulphide from the synthesis mixture by washing the reaction products with ether.

### Brief description of the drawings

Figure 1 shows an HPLC diagram with a characteristic total alkaloid composition of the roots of *Chelidonium majus L.*
Figure 2 shows the HPLC fingerprint of a preparation according to Example 1.
Figure 3 shows selected phosphorus derivatives suitable as reagents.

### Description of the invention

It is the object of the present invention to provide a novel process for the preparation of a reaction product of alkaloids with phosphorus derivatives, which process promotes the conversion of the alkaloids into a water-soluble form. In particular, it is intended thereby that, in addition to selected alkaloids, the total alkaloids of *Chelidonium majus L.* also be convertible and a pharmaceutical preparation of low toxicity and having as broad an action spectrum as possible be obtainable.

This object is achieved by the features of Claim 1. Further embodiments and developments of the invention are shown in the subclaims.

The process according to the invention comprises reacting an alkaloid or a mixture of alkaloids contained in *Chelidonium majus L.* in an organic solvent with a phosphorus derivative and then washing the reaction product with water. The phosphorus derivative preferably contains at least one aziridine group. The washed reaction product can then be converted into a water-soluble form. Particularly if it is toxic, the phosphorus derivative is preferably water-soluble.

As a result of washing with water, it is now possible substantially to simplify the preparation process since complicated safety precautions owing to the risk of explosion of the ether no longer need be taken. Consequently, the process can also be carried out without problems on an industrial scale. Moreover, the composition of the reaction product, which is decisive for the pharmacological action, is changed by the washing process since, with the liquid-liquid partition, water-soluble components are dissolved out of the reaction product and are removed. Surprisingly, it has now been found that the water additionally has a catalytic effect in relation to the reaction products and thus influences the structure and composition of the product obtained from the synthesis in that 10 to 15 times the amount of the reaction product can be converted into a water-soluble form than if it was washed with an organic solvent.

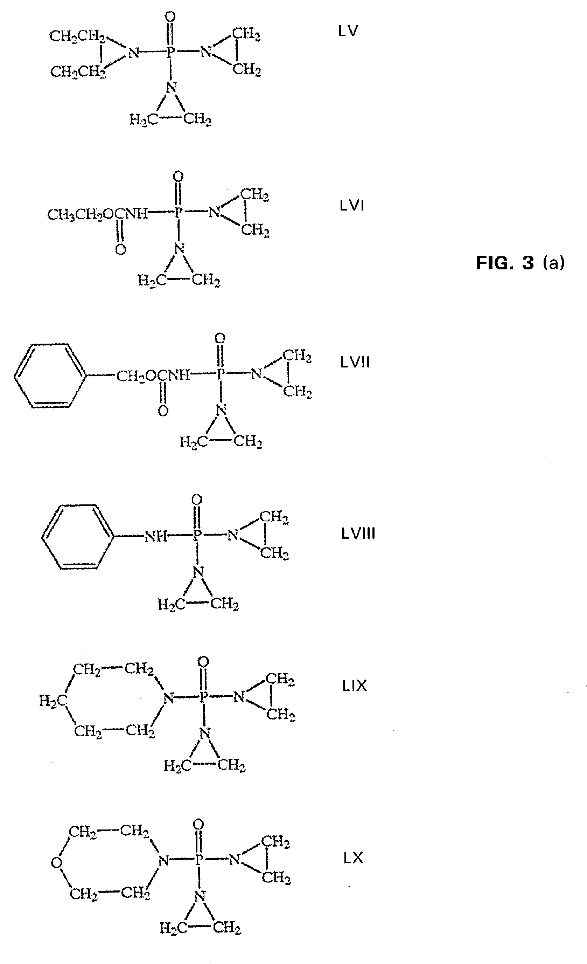

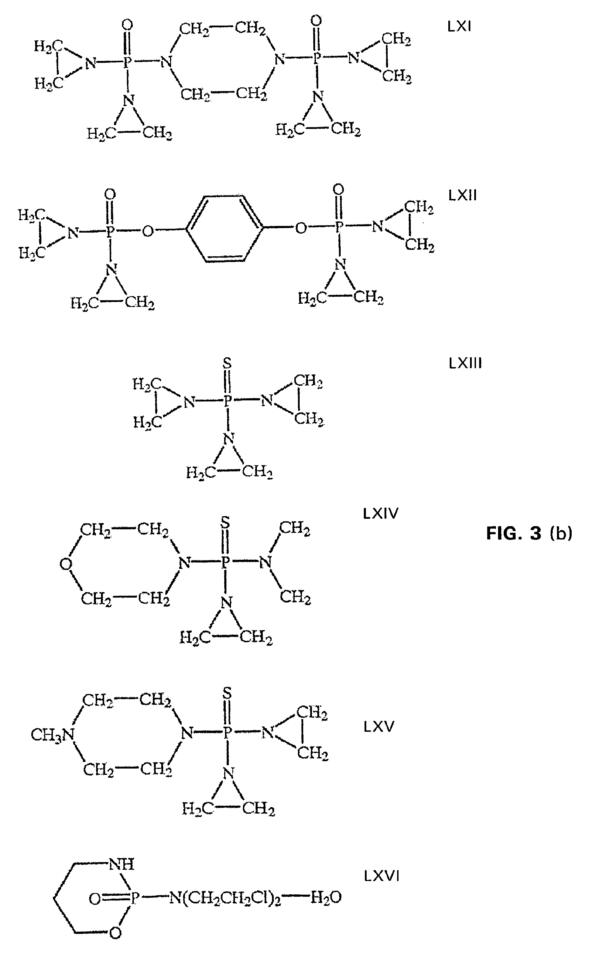

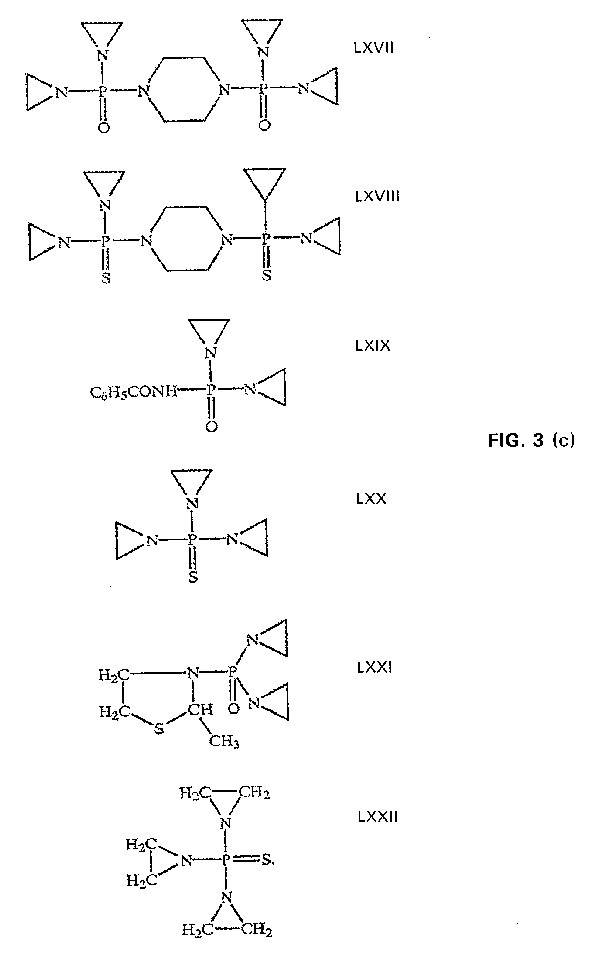

Washing with water converts the reaction product into a state which substantially facilitates subsequent conversion into a water-soluble form. This measure is suitable for alkaloids of *Chelidonium majus L*.. The present process can be used, for example, for reactions of alkaloids with the carcinostatic phosphorus compounds, as mentioned in Claim 1 of AT 377 988, the phosphorus derivatives shown in Figure 3, in particular those having an aziridine group, being particularly suitable.

A suitable organic solvent is any agent in which the alkaloids intended for the reaction are soluble. The alkaloids can, for example, be dissolved in dichloromethane or chloroform.

The reaction of the alkaloids take place at elevated temperature, preferably at the boiling point of the solvent.

The reaction product is preferably converted into a water-soluble form after washing with water. This can be carried out according to the process described in AT 377 988 and AT 354 644, by conversion into the water-soluble salts, in particular into the hydrochlorides, for example by passing in HCl gas or adding an HCl solution to the organic solution of the washed reaction product, after which the hydrochlorides are precipitated. The water-soluble salt of the reaction product is suitable for application in injection solutions.

In an embodiment of the invention, the reaction is carried out with tris(1-aziridinyl)phosphine sulphide (CAS No. 52-24-4), which in the pharmacopoeia is also known as thiotepa. Further synonyms are ledertepa, Onco thiotepa, TESPA, tespamine, thiophosphamide, thio-TEPA, thiotriethylenephosphoramide, tifosyl, triaziridinylphosphine sulphide, N,N',N"-tri-1,2-ethanediylphosphorothioine triamide; N,N',N"-tri-1,2-ethanediylthiophosphoramide, tri-(ethyleneimino)thiophosphoramide; N,N',N"-triethylenethiophosphoramide, triethylenethiophosphoro-triamide, m-triethylenethiophosphoramide, m-tris(aziridin-1-yl)phosphine sulphide, triethylenethiophosphoramide, tris(1-aziridinyl)phosphine sulphor, tris (ethylene-imino)thiophosphate, TSPA and WR 45312.

In a further embodiment of the invention, the extract of alkaloids, optionally the total alkaloids, of *Chelidonium majus L.* in an organic solvent is reacted with tris(1-aziridinyl)-phosphine sulphide, and the resulting reaction product, optionally present as a complex, is then washed at least once with water. Since the tris (1-aziridinyl)phosphine sulphide decomposes in water, the unconverted residue of tris(1-aziridinyl)phosphine sulphide present in excess after the reaction can be removed from the organic phase by this measure. Preferably, the organic solution containing the reaction product is washed several times and each time is saturated with water. Particularly preferable, the washing is repeated until the excess of highly toxic tris(1-aziridinyl)phosphine sulphide has been completely removed from the reaction product.

In addition, toxic alkaloids which contribute to adverse reactions in medical applications and might even cause cirrhosis of the liver are removed with the aqueous phase from the synthesis mixture or their concentrations are reduced. By means of the Ames test, it was shown that the reaction product of this embodiment, prepared according to the invention, is not mutagenic.

This reaction product is a complex mixture of alkaloids with higher molecular weight reaction products of tris(1-aziridinyl)phosphine sulphide with alkaloids and of degradation products of tris(1-aziridinyl)phosphine sulphide. As a result of the synthesis process, the solubilities of the alkaloids change. The concentration of tertiary alkaloids is increased while the one of quaternary alkaloids is decreased. The reaction product consists of a complex of about 60 to 70% of Chelidonium alkaloids with about 30 to 40% of reaction products of tris(1-aziridinyl)phosphine sulphide.

Tertiary Chelidonium alkaloids represent the main part of the components. For example, the following tertiary alkaloids may be contained in the synthesis mixture: chelidonine, protopin, stylopin, allocryptopin, α-homochelidonine, chelamidine, chelamine, L-sparteine, chelidimerine, dihydrosanguinarine, oxysanguarine, oxychelidonine and methoxychelidonine.

Quaternary alkaloids (e.g. berberine) are substantially removed with the water from the synthesis mixture by the liquid-liquid partition. Under the stated reaction conditions, berberine furthermore forms no compounds with tris(1-aziridinyl)phosphine sulphide.

The reaction product of this embodiment also shows a better medical action spectrum than a reaction product washed with ether. It destroys cancer cells by apoptosis but - in contrast to most known cytostatic agents - without also attacking healthy cells. This results in the good tolerance of a therapy with this preparation and its general suitability for prophylactic use. It is simple to handle and has no significant adverse reactions in therapeutic doses. The interplay of the substances in the synthesis mixture is responsible for the medical effect.

The reaction product of the total alkaloids of *Chelidonium majus L.* exhibits biological activity in regulation of the metabolism and is suitable for the prevention and therapy of metabolic diseases, such as osteoporosis, but also rheumatic diseases, allergies, viral infections, epilepsy, multiple sclerosis, scars, skin tumours and postoperative wounds.

An extract of the dried roots of *Chelidonium majus L.* is preferably used as a starting material for the synthesis. The roots have a higher content of alkaloids than the leaves or the stem.

The customary pharmaceutical excipients, in particular for solutions, for example injection or infusion solutions, or for ointment, compress or suspensory bases, are suitable for drugs which contain the reaction products prepared according to the invention.

The following examples illustrate the invention:

### Example 1

### A) Extraction of the alkaloids:

a. 25 g of an alkaloid salt mixture are suspended in water and transferred to a separating funnel. After the addition of 100 ml of dichloromethane, the separating funnel is shaken. The organic phase is then separated off and is filtered into a glass bottle.
b. 1 N NaOH (pH 8-9) is added to the aqueous phase until turbidity occurs. After the addition of 100 ml of dichloromethane, the mixture is shaken. The organic phase is then separated off and is combined with the dichloromethane phase from step a. This process is repeated, for example 3 times. The organic phases are filtered and combined.
c. The aqueous phase is adjusted to a pH of 10 by adding NaOH. After the addition of dichloromethane, the mixture is shaken. The organic phase is then separated off, filtered and mixed with the other organic phases. The aqueous phase is now adjusted to a pH of 13 with NaOH and the extraction is repeated with dichloromethane.
d. The combined organic phases are evaporated to give an oily, brown material.

### B) Reaction with tris(1-aziridinyl)phosphine sulphide:

The alkaloid residue is dissolved in dichloromethane, and tris(1-aziridinyl)phosphine sulphide is added. The mixture is refluxed at 80°C for 2 hours. After cooling to room temperature, the reaction mixture is clarified. Filtration is then carried out and the filtrate is washed several times, for example 3 times or more, with 250 ml of water in a separating funnel.

### C) Reaction with HCl

The washed solution is transferred to a glass beaker, stirred and saturated with HCl gas, a hydrochloride complex being precipitated. The precipitated product is filtered off and is washed with diethyl ether, dried and then dissolved in water.

In rats, an LD₅₀ value of 485 mg/kg was determined from the reaction product according to Example 1. Studies in mice and rats showed that the product according to the invenmtion modulates the hormone regulation of the thymus and induces the synthesis of substances having thymosin-like activity in animals whose thymus has been removed. This effect is dose-dependent. The preparation increases the number of T-lymphocytes in the peripheral blood by up to 50% (4.04 ± 0.43 x 10⁹/l before the treatment, 6.24 ± 0.73 x 10⁹/l after the treatment), modulates the humoral immune response to penetrating antigen and the natural killer cell activity of the spleen cells (198.20 ± 17.69% compared with 71.50 ± 9.10% in the control group) and enhances the interferon liberation potential of the white blood corpuscles in animal experiments. The results of the animal experiments are confirmed by clinical observations. Thus, the improvement in the immune parameters was also observed in cancer patients.

Doses of about 5 mg of the preparation from Example 1 per 70 kg body weight can be used for prophylactic and immunological applications. For cancer treatment, 5 mg of the preparation per 20 kg body weight are preferably administered.

### Example 2: HPLC fingerprints

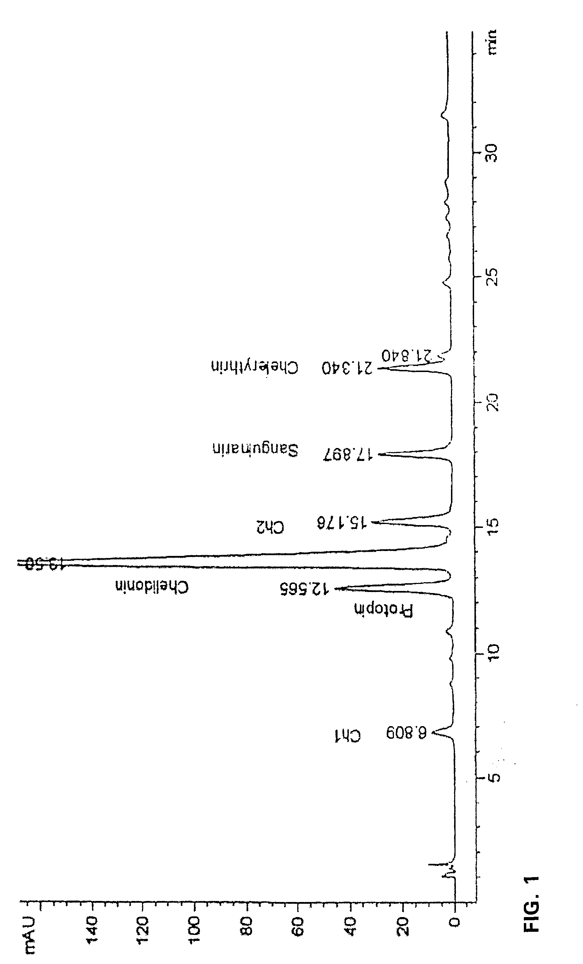

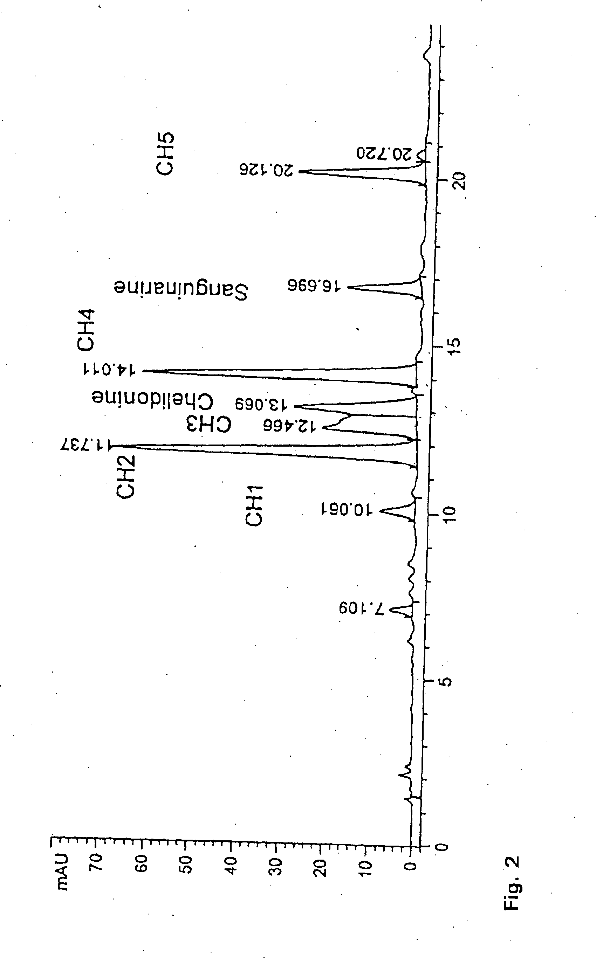

The determination was carried out by ion pair reverse-phase chromatography in the gradient mode and with spectral measurement using a DAD detector at 285 nm. At the same time, chromatograms were prepared using reference alkaloids. In addition, an HPLC-MSD analysis was carried out, which showed that there were no peaks apart from those of the alkaloids. The HPLC diagrams of Figures 1 and 2 were obtained on the basis of the following experimental data:
Chromatography parameters:
   Column: LiChrospher 60 RP Select B, 5 µm, 125 x 24 mm ID
   Eluent: A) 200 ml (acetonitrile) + 800ml (water) + 1.5 g (hexanesulphonic acid) + 0.3 ml (85% phosphoric acid)
   B) 900 ml (acetonitrile) + 100 ml (water) + 1.5 g (hexanesulphonic acid) + 0.3 ml (85% phosphoric acid)
   Gradient: 5 min isocratically 100% A;
      up to 40% B in 24 min
      up to 100% B in 1 min
      5 min 100% B;
      5 min equilibration with 100% A
   Detection: UV light at 285 nm
   Eluate flow rate: 1 ml/min, stop after 35 min.
   Injected volume: 10 µl

### Sample preparation:

Extract before reaction (Figure 1): 25 mg of alkaloids are dissolved in 40 ml of methanol by ultrasonics, made up to 50 ml and filtered through a membrane filter.

Reaction product (Figure 2): The reaction product is converted into the hydrochloride salt, dissolved in water in a concentration of 1 mg/ml and adjusted to a pH of between 2.5 and 6.5.

The following examples show various applications of the compound (which in the following is called Ukrain^{®}) resulting from the procedure described in Example 1.

### Example 3: Osteoporosis type I and II:

30 patients with type I and II osteoporosis were treated with Ukrain^{®}. 5 mg doses in a concentration of 1 mg/ml were administered intravenously, in each case once a week over 10 weeks. The progress of the therapy was monitored by determination of haematological and biochemical parameters and measurement of serum FSH, LH and prolactin concentrations. Calcium and phosphorus were measured in serum and urine. After treatment for three months, an improvement in the clinical picture and increased physical activity were found in the case of all patients. No adverse reactions of the treatment were observed.

### Example 4: Osteogenesis imperfecta:

Type I Osteogenesis imperfecta was diagnosed in a 14 year old female patient. X-ray pictures indicated diffuse osteoporosis, and blue sclera, milky teeth (dentiogenesis imperfecta), conical thorax, scoliosis and muscular hypotension occurred. The patient was administered Ukrain^{®} intravenously in a dose of 5 mg once a week. Three therapy cycles with a three-month pause between the cycles were carried out. The treatment achieved a significant improvement in muscle function; sclera and teeth became white again. The scoliosis improved and the extent of the therapeutic physical exercises could be markedly increased owing to the higher load capacity. The patient's body became well proportioned again. The body weight and the body size were now normal for the age. X-ray pictures indicated the restoration of the osseous structure; there were no longer any detectable signs of osteoporosis in the X-ray pictures.

### Example 5: Spondylarthritis:

A 71 year old female patient complained about severe pains in the lower back region, the L₁ to L₃ region, with restrictions of mobility. The patient was treated with Ukrain^{®} in a 5 mg dose administered intravenously once a week, as well as with compresses applied superficially over four weeks. The patient's condition improved. Mobility was completely restored.

### Example 6: Ulcer-forming skin lesion:

A seven year old boy had a large ulcer-forming postoperative skin lesion in the left region of the scapula. The scar was too large for the use of plastic surgery. The patient was treated superficially with compresses containing Ukrain^{®} over 12 weeks. The scar improved significantly. The ulcer receded and furthermore no inflammation developed.

### Example 7: Asthmatic allergy

A severe allergy with coughing and asthmatic attacks was observed in a 12 year old male patient. Treatment was carried out with Ukrain^{®} in a daily dose of 5 mg over 3 weeks. His condition then improved dramatically and the patient is now considered to have been cured.

### Example 8: Milk allergy

A 12 year old male patient suffered from a severe milk allergy, allergic attacks also being caused by food containing milk, e.g. chocolate. The patient was administered Ukrain^{®} intramuscularly every second day for 2 weeks. His condition improved significantly. The patient is now considered to have been cured.

### Example 9: Allergy to cats

A 9 year old female patient suffered from an allergy to cats which caused severe asthmatic attacks. The patient was treated with Ukrain^{®} in a dose of 5 mg twice a week. Her condition improved and she is now considered to have been cured.

### Example 10: Herpes zoster

A 64 year old female patient was diagnosed with a Herpes zoster infection which recurred. Typical blisters along a tear in the Th₃-Th₄ region with ulceration was found. Ukrain^{®} was administered intravenously in a dose of 5 mg twice a week over 8 weeks. No recurrence has been observed for 4 years.

### Example 11: Hepatitis C

A 42 year old male patient with chronic Hepatitis C, HCV-RNA positive and HCV genotype 1 b complained about tiredness, accompanying pain and poor physical powers. He was administered Ukrain^{®} intravenously in a dose of 5 mg twice a week over 5 weeks. As a result of the treatment, the patient was freed from the pain, his general condition improved and his physical powers increased. The patient is now HCV-RNA negative.

### Example 12: Allergy

Patient A.N., 12 years old, had severe allergic attacks with coughing, including asthmatic episodes. He was treated with Ukrain^{®} administered orally, 5 mg daily. After 4 weeks he showed a dramatic improvement, and no more attacks occurred.

### Example 13: Postoperative wound (skin lesion with ulceration)

Patient S.D., 7 years old, had a large skin lesion in the region of the left shoulder following an operation. The skin lesion was too large for plastic reconstruction. The patient was treated locally with Ukrain^{®}-compresses, 3 months. The skin lesion has substantially reduced in size. No ulcer, no inflammation.

### Example 14: Osteoporosis

Patient H.J., 68 years old, had difficulties in walking, fatigue. After thorough clinical and laboratory investigation, osteoporosis type I was diagnosed. After a 10 weeks' treatment with Ukrain^{®}, 5 mg intravenously per week, the patient felt a subjective improvement, confirmed by significant changes in the laboratory results regarding bone density, calcium and hormone level.

### Example 15: Shingles

Patient K.R., 37 years old, having had chicken pox at the age of 7 years, now showed typical painful skin eruptions along the ribs - shingles (Herpes zoster). After treatment with 5 mg Ukrain^{®} intravenously, twice a week during 4 weeks, no recurrence occurred for 6 years.

### Example 16: Hepatitis B

Patient G.H., 48 years old, showed weakness, tiredness and decrease in vitality. Diagnosis after thorough clinical and laboratory investigation: chronic Hepatitis B, HBₛ-Ag positive, increased enzyme levels in the blood. After treatment with 5 mg intravenously Ukrain^{®} once a week during 3 months, a clear subjective improvement occurred and the vitality increased. A significant decrease in viral load and a normalization of enzyme levels was shown in laboratory tests.

### Example 17: Rheumatic diseases, osteoarthritis

Patient I.N., 71 years old, had severe back pain in the region L1-L3, with limited mobility. Diagnosis after thorough clinical and radiological investigation: osteoarthritis (arthrosis). Treatment: Ukrain^{®}, 5 mg intravenously twice a week, during 6 weeks. The pain was clearly reduced, and the patient achieved an increased range of mobility compared with the reference range.

### Example 18: Epileptic attacks

Patient D.R., 46 years old, had complex focal epileptic attacks with the tendency to deterioration of the symptoms. High doses of phenytoin with adenopathy as a side effect. After treatment with 10 mg Ukrain^{®} intravenously twice a week during 6 weeks, a considerable reduction in symptoms was noted, the attacks have become rarer and less pronounced. Much lower doses of antiepileptic drugs could be applied.

### Example 19: Multiple sclerosis

Patient M.G., 36 years old, felt unexplainable weakness in the legs, and subsequently giddiness. Intervals between episodes became shorter. Multiple sclerosis has been diagnosed after thorough investigation. A physical therapy was used without success. After a treatment with 5 mg Ukrain^{®} intravenously twice a week during 4 months, an increase in the length of intervals between attacks was noted, the patient felt more power in the legs and no more giddiness.

### Example 20: Scars

Patient I.N. had a large hard scar in the abdominal region after an abdominal operation. After treatment with 5 mg Ukrain^{®} intravenously twice a week for 4 weeks and locally as compresses, a clear positive cosmetic effect was noted.

### Example 21: Influenza

Patient B.N., 24 years old, had annual influenza infections, in some cases with pneumonia. Ukrain^{®} was used as a prophylactic measure: 5 mg intravenously twice a week, total dose 50 mg in the period before outbreak of the epidemic. The patient is now four years without disease.

### Example 22: Diabetes mellitus type II

Patient B.L., 62 years old, had diabetes mellitus type II (non-insulin-dependent diabetes), was treated with a daily dose of 10 mg of glipizide (antidiabetic drug of the sulphonylurea group). The patient showed high blood pressure, overweight in spite of diet, weariness, quick fatigue and low vitality. After treatment with 5 mg Ukrain^{®} intravenously twice a week during 2 months, the general condition clearly improved, vitality increased, the body weight reduced. Glipizide was gradually discontinued and the patient now feels well.

### Example 23: Rehabilitation

Patient O.L., 74 years old, had a left hip joint fracture. After a major operation lasting several hours with joint implantation, the patient was confined to bed. After treatment with 5 mg Ukrain^{®} intravenously twice a week during 6 weeks, the general condition clearly improved, the rehabilitation period was significantly shortened.

### Example 24: Allergy to chocolate

Patient V.P., 8 years old, was allergic to chocolate. After treatment with 5 mg Ukrain^{®} administered orally 3 times a week for 4 weeks, no further allergic attacks occurred.

### Example 25: Rehabilitation

Patient J.R., 78 years old, had a surgery for gallstones and cholecystitis (cholecystectomy). After the operation, Ukrain^{®} was administered orally, 5 mg 3 times a week for 2 weeks. Rapid and trouble-free recovery as noted, without any complications. The patient was discharged from hospital sooner than expected.

All the applications shown in examples 3 to 25 may also be successfully performed - if even in some cases with slightly less efficiency - with the compounds described in AT 377 988.

## Claims

1. A process for reacting alkaloids, comprising:
a) preparing a reaction product by reacting at least one alkaloid selected from the alkaloids which are contained in *Chelidonium majus L.* with a phosphorus derivative in organic solution at elevated temperature, in particular at the boiling point of the solvent, wherein said phosphorous derivative contains at least one aziridine group; and
b) washing the reaction product obtained in step a); **characterized in that** the reaction product is washed with water.

2. The process according to Claim 1, the phosphorus derivative being tris(1-aziridinyl)phosphine sulphide (CAS 52-24-4).

3. The process according to Claim 1, wherein a mixture of alkaloids is reacted.

4. The process according to Claim 1, wherein the solvent is dichloromethane.

5. The process according to Claim 1, wherein the reaction products are converted into a salt, in particular a water-soluble salt, after the washing step.

6. The process according to Claim 5, the salt being a chloride.

7. An alkaloid reaction product obtainable in a process according to any one of Claims 1 to 6, wherein the reaction product present after the washing step with water
a) comprises a derivatized alkaloid obtained from reaction of a tertiary alkaloid selected from the alkaloids which are contained in *Chelidonium majus L.* with a phosphorus derivative that contains at least one aziridine group;
b) is of a nature such as to significantly facilitate, preferably by a factor of 10 to 15, a subsequent conversion into a water-soluble salt, relative to an alkaloid reaction product washed with an organic solvent; and
c) has a reduced amount of or is free from water-soluble toxic alkaloid or phosphorous derivative decomposition compounds.

8. An alkaloid reaction product according to Claim 7, which contains a derivatized alkaloid in a water-soluble form, preferably as a hydrochloride.

9. The alkaloid reaction product according to Claim 7 or 8, wherein at least one alkaloid is selected from the group consisting of chelidonine, protopin, stylopin, allocryptopin, homochelidonine, sanguinarine, chelamidine, chelamine, L-sparteine and oxychelidonine.

10. The alkaloid reaction product according to any one of Claims 7 to 9 for use as a drug.

11. Use of an alkaloid reaction product defined in any one of Claims 7 to 9 in the manufacture of a preparation for the prophylaxis or treatment of conditions, diseases or organic malfunctions selected from the group consisting of cancer, immune diseases, metabolic diseases, allergies, osteoporosis, viral infections, skin tumours, rheumatic diseases, epilepsy and multiple sclerosis.

12. Use of an alkaloid reaction product defined in any one of Claims 7 to 9 in the manufacture of a preparation for the treatment of scars and postoperative wounds.

## Patentansprüche

1. Ein Verfahren zur Reaktion von Alkaloiden, umfassend:
a) Herstellen eines Reaktionsprodukts durch Reaktion mindestens eines Alkaloids, ausgewählt aus den in *Chelidonium majus L.* enthaltenen Alkaloiden, mit einem Phosphorderivat in organischer Lösung bei erhöhter Temperatur, insbesondere beim Siedepunkt des Lösungsmittels, wobei das Phosphorderivat mindestens eine Aziridingruppe enthält; und
b) Waschen des in Schritt a) erhaltenen Reaktionsprodukts; **dadurch gekennzeichnet, dass** das Reaktionsprodukt mit Wasser gewaschen wird.

2. Verfahren gemäß Anspruch 1, wobei das Phosphorderivat Tris (1-aziridinyl) phosphinsulfid (CAS 52-24-4) ist.

3. Verfahren gemäß Anspruch 1, wobei ein Gemisch von Alkaloiden zur Reaktion gebracht wird.

4. Verfahren gemäß Anspruch 1, wobei das Lösungsmittel Dichlormethan ist.

5. Verfahren gemäß Anspruch 1, wobei die Reaktionsprodukte nach dem Waschschritt in ein Salz, insbesondere in ein wasserlösliches Salz, umgesetzt werden.

6. Verfahren gemäß Anspruch 5, wobei das Salz ein Chlorid ist.

7. Alkaloidreaktionsprodukt, das in einem Verfahren gemäß einem der Ansprüche 1 bis 6 erhalten werden kann, wobei das im Anschluss an den Waschschritt mit Wasser vorliegende Reaktionsprodukt
a) ein derivatisiertes Alkaloid umfasst, erhalten aus der Reaktion eines tertiären Alkaloids, ausgewählt aus den in *Chelidonium majus L.* enthaltenen Alkaloiden, mit einem mindestens eine Aziridingruppe enthaltenden Phosphorderivat;
b) so beschaffen ist, dass es eine anschließende Umsetzung in ein wasserlösliches Salz signifikant begünstigt, bevorzugt um einen Faktor von 10 bis 15, bezogen auf ein mit einem organischen Lösungsmittel gewaschenes Alkaloidreaktionsprodukt; und
c) einen verringerten Anteil an wasserlöslichen, toxischen Alkaloiden oder Abbauprodukten von Phosphorderivaten aufweist bzw. frei von diesen ist.

8. Alkaloidreaktionsprodukt gemäß Anspruch 7, das ein derivatisiertes Alkaloid in einer wasserlöslichen Form, bevorzugt als ein Hydrochlorid, enthält.

9. Alkaloidreaktionsprodukt gemäß Anspruch 7 oder 8, wobei mindestens ein Alkaloid ausgewählt ist aus der Gruppe bestehend aus Chelidonin, Protopin, Stylopin, Allocyptopin, Homochelidonin, Sanguinarin, Chelamidin, Chelamin, L-Spartein und Oxychelidonin.

10. Alkaloidreaktionsprodukt gemäß einem der Ansprüche 7 bis 9 zur Verwendung als ein Arzneimittel.

11. Verwendung eines Alkaloidreaktionsprodukts, wie in einem der Ansprüche 7 bis 9 definiert, bei der Herstellung einer Zubereitung für die Prophylaxe oder Behandlung von Beschwerden, Erkrankungen oder organischen Funktionsstörungen ausgewählt aus der Gruppe bestehend aus Krebs, Immunerkrankungen, Stoffwechselerkrankungen, Allergien, Osteoporose, Virusinfektionen, Hauttumoren, rheumatischen Erkrankungen, Epilepsie und multipler Sklerose.

12. Verwendung eines Alkaloidreaktionsprodukts, wie in einem der Ansprüche 7 bis 9 definiert, bei der Herstellung einer Zubereitung für die Behandlung von Narben und postoperative Wunden.

## Revendications

1. Un procédé pour mettre en réaction des alcaloïdes, comprenant :
a) la préparation d'un produit de réaction par mise en réaction d'au moins un alcaloïde sélectionné parmi les alcaloïdes contenus dans *Chelidonium majus L.* avec un dérivé de phosphore en solution organique à une température élevée, en particulier au point d'ébullition du solvant, dans lequel ledit le dérivé phosphoreux contient au moins un groupe aziridine ; et
b) le lavage du produit de réaction obtenu en étape a) ; **caractérisé en ce que** le produit de réaction est lavé avec de l'eau.

2. Le procédé selon la revendication 1, le dérivé de phosphore étant du sulfure de tris(1-aziridinyl) phosphine (CAS 52-24-4).

3. Le procédé selon la revendication 1, dans lequel un mélange d'alcaloïdes est mis en réaction.

4. Le procédé selon la revendication 1, dans lequel le solvant est le dichlorométhane.

5. Le procédé selon la revendication 1, dans lequel les produits de réaction sont convertis en un sel, en particulier un sel soluble dans l'eau, après l'étape de lavage.

6. Le procédé selon la revendication 5, le sel étant un chlorure.

7. Un produit de réaction d'alcaloïde susceptible d'être obtenu dans un procédé selon l'une quelconque de revendications 1 à 6, dans lequel le produit de réaction, présent après l'étape de lavage avec de l'eau,
a) comprend un alcaloïde dérivatisé, obtenu à partir de la réaction d'un alcaloïde tertiaire sélectionné parmi les alcaloïdes qui sont contenus dans le *Chelidonium majus L.* avec un dérivé de phosphore qui contient au moins un groupe aziridine ;
b) est d'une nature apte à faciliter de manière significative, de préférence par un facteur de 10 à 15, une conversion subséquente en un sel soluble dans l'eau, relativement à un produit de réaction d'alcaloïde lavé avec un solvant organique ; et
c) présente une quantité réduite de, ou est exempt de, l'alcaloïde toxique soluble dans l'eau ou des composés de décomposition dérivés phosphoreux.

8. Un produit de réaction d'alcaloïde selon la revendication 7, qui contient un alcaloïde dérivatisé sous une forme soluble dans l'eau, de préférence sous forme d'un hydrochlorure.

9. Le produit de réaction d'alcaloïde selon la revendication 7 ou 8, dans lequel au moins un alcaloïde est sélectionné dans le groupe constituté de la chélidonine, la protopine, la stylopine, l'allocryptopine, l'homochélidonine, la sanguinarine, la chélamidine, la chelamine, la L-sparteine et l'oxychélidonine.

10. Le produit de réaction d'alcaloïde selon l'une quelconque des revendications 7 à 9, pour utilisation comme médicament.

11. Utilisation d'un produit de réaction d'alcaloïde défini selon l'une quelconque des revendications 7 à 9 dans la fabrication d'une préparation pour la prophylaxie ou le traitement de conditions, maladies ou dysfonctionnements organiques sélectionnés dans le groupe constitué du cancer, des maladies immunes, des maladies métaboliques, des allergies, de l'ostéoporose, des infections virales, des tumeurs de peau, des maladies rhumatismales, de l'épilepsie et de la sclérose en plaques.

12. Utilisation d'un produit de réaction d'alcaloïde défini selon l'une quelconque de revendications 7 à 9 dans la fabrication d'une préparation pour le traitement des cicatrices et des blessures postopératoires.
